# EUROPÄISCHE PATENTSCHRIFT

(11) **EP 1 721 591 B1**
(45) Veröffentlichungstag und Bekanntmachung des Hinweises auf die Patenterteilung: **21.04.2010**
(21) Anmeldenummer: 06005370.9
(22) Anmeldetag: 16.03.2006
(51) Int. Cl.: A61F 13/62

(54) **Verschlussstreifen für Windeln**
Diaper fastener
Dispositif de fermeture pour des couches-culottes

(30) Priorität: 12.04.2005 DE 102005016895
(43) Veröffentlichungstag der Anmeldung: 15.11.2006
(73) Patentinhaber: Nordenia Deutschland Gronau GmbH, 48599 Gronau (DE)
(72) Erfinder: Baldauf, Georg, 48366 Laer (DE); Schönbeck, Marcus, 33775 Versmold (DE)
(74) Vertreter: Albrecht, Rainer Harald

(56) Entgegenhaltungen:
- EP-A- 0 809 992
- US-A- 3 800 796
- US-A- 4 381 781
- US-A- 5 352 518
- US-B1- 6 225 243

## Beschreibung

Die Erfindung betrifft einen Verschlussstreifen für Windeln mit
einem Mittelteil, der beim Ziehen des Verschlussstreifens in Streifenlängsrichtung elastisch ist,
einem unelastischen Endteil mit einem Hakenelement und
einem unelastischen Anschlussteil zur Befestigung an einer Windel,
wobei das Endteil und das Anschlussteil an dem Mittelteil befestigt sind. Die Streifenlängsrichtung erstreckt sich vom Anschlussteil zum Endteil.

Der Verschlussstreifen ist Teil eines mechanischen Klettverschlusses an einer Windel. Ein Teil des Klettverschlusses ist mit weiblichen Verschlusselementen versehen und im vorderen Bündchenbereich der Windel aufgeklebt. Das zugehörige Hakenelement ist an dem beschriebenen Verschlussstreifen angebracht, der am hinteren Bündchenbereich der Windel befestigt ist.

Ein Verschlussstreifen für Windeln mit den beschriebenen Merkmalen ist aus EP 0 809 992 B1 bekannt. Das Mittelteil des Verschlussstreifens besteht aus einer plastisch verformbaren Folie, auf die gerade oder spiralförmig angeordnete Streifen aus einem elastomeren Material aufgebracht sind. Vor dem erstmaligen Gebrauch weist der Verschlussstreifen keine Elastizität auf. Der Verschlussstreifen wird elastisch, wenn er beim erstmaligen Gebrauch durch Ziehen in der Längsrichtung des Streifens gedehnt wird. Hierbei besteht die Gefahr, dass beim Schließen des Windelverschlusses oder beim Tragen der Windel die elastischen Verschlussstreifen überdehnt werden. Die Überdehnung ist mit einer bleibenden Dehnung des Materials verbunden und wirkt sich nachteilig auf die Passform der Windel aus.

Vor diesem Hintergrund besteht die der Erfindung zugrundeliegende Aufgabe darin, einen Verschlussstreifen für Windeln anzugeben, bei dem eine Überdehnung durch unsachgemäßen Gebrauch oder durch Beanspruchungen während eines bestimmungsgemäßen Gebrauchs weitgehend ausgeschlossen ist.

Ausgehend von einem Verschlussstreifen mit den eingangs beschriebenen Merkmalen wird die Aufgabe erfindungsgemäß dadurch gelöst, dass das Mittelteil eine Schicht aus einem elastischen Spinnvlies aufweist, welches bevorzugte Dehnungseigenschaften in Streifenlängsrichtung besitzt, wobei das Spinnvlies aus Filamenten besteht, die einen Filamentkern aus einem thermoplastischen Elastomer und einen durch Dehnung des Spinnvlieses verstreckten Filamentmantel aus einen nichtelastischen thermoplastischen Polymer aufweisen, und wobei das Mittelteil (1) bis zu einer durch die Vorverstreckung der nichtelastischen Filamentanteile des Spinnvlieses festgelegten Dehngrenze elastisch dehnbar ist und die Dehngrenze durch einen starken Anstieg der Zurweiteren Dehnung benötisten Kraft wahrnehmbar ist.

Der erfindungsgemäße Verschlussstreifen zeichnet sich durch eine deutlich wahrnehmbare Dehngrenze aus. Das Mittelteil des Verschlussstreifens kann bis an diese Grenze mit geringer Kraft gedehnt werden und stellt sich bei Entlastung wieder elastisch zurück. Bei der Benutzung des Verschlussstreifens als Windelverschlusslasche wird dieser Bereich als elastischer Arbeitsbereich wahrgenommen. Bei Erreichen der durch das Material festgelegten Dehngrenze ist ein starker Anstieg der zur weiteren Verdehnung nötigen Kraft zu bemerken, und der Windelverschluss verhält sich oberhalb der definierten Dehngrenze unelastisch. Die Eigenschaft des erfindungsgemäßen Verschlussstreifens wirkt sich auch vorteilhaft auf die Passform der Windel aus. Durch die Elastizität der Windelverschlusslasche bzw. des Verschlussstreifens kann der Windelverschluss den Bewegungen der Person entsprechend gedehnt werden und sich wieder entspannen. Dabei wird der Windelverschluss aufgrund der vorliegenden Dehngrenze nicht überdehnt, so dass die Passform der Windel während des Gebrauchs erhalten bleibt. Die prozentuale Auslängung des Mittelteils bis zum Erreichen der Dehngrenze wird durch die Vorverstreckung der nichtelastischen Filamentanteile des Spinnvlieses festgelegt.

Die Filamente des Spinnvlieses sind als sogenannte Bikomponentenfasern aufgebaut. Geeignete polymere Mischungen für den elastomeren Filamentkern umfassen beispielsweise Polyurethane, Styrol-Blockcopolymere, Polyethylene, Polyethylen-Copolymere und Polypropylen-Copolymere sowie Mischungen aus diesen Polymeren als elastische Komponente. Der nichtelastische Filamentmantel kann beispielsweise aus Polypropylen oder Polyethylen bestehen. Der Spinnvliesstoff wird aus Endlosfilamenten hergestellt, die mittels eines Luftstromes unter Ausnutzung eines Venturieffektes aus Spinndüsen abgezogen und in Wirrlage auf einem Spinnband abgelegt werden. Das Faservlies wird verfestigt, wobei die Verfestigung mittels eines Kalanders unter Einfluss von Wärme und Druck, durch Vernadeln, Heißluftverfestigung oder andere, dem Fachmann zur Vliesverfestigung bekannte Verfahren erfolgen kann. Eine nachfolgende Verdehnung des verfestigten Faservlieses in einer Richtung, die der Streifenlängsrichtung entspricht, bewirkt eine Kaltverstreckung der nichtelastischen Filamentanteile. Die Schicht des Mantelpolymers wird in Filamentrichtung orientiert, wobei das teilweise bis zur Reißgrenze orientierte Mantelpolymer für einen steilen Anstieg der Reißkraftwerte der Bikomponentenfasern sorgt. Nach der Dehnung stellt sich das Faservlies unter der Wirkung des elastischen Kerns der Bikomponentenfasern elastisch zurück. Durch die beschriebene Vordehnung wird ein elastischer Spinnvliesstoff mit definierter Dehngrenze erzeugt, welcher sich nach Maßgabe der Vordehnung bis zu einer deutlich wahrnehmbaren Dehngrenze elastisch dehnen lässt.

Das Mittelteil kann als einschichtiges Element ausschließlich aus einem Spinnvlies bestehen, wobei das Spinnvlies vorzugsweise ein Flächengewicht zwischen 80 g/m² und 200 g/m² aufweist.

Eine weitere, unter die erfindungsgemäße Lehre fallende Ausführung sieht vor, dass das Mittelteil eine elastische Trägerfolie aufweist, auf die das Spinnvlies ein- oder beidseitig aufkaschiert ist. Auch die Trägerfolie besitzt bevorzugte Dehnungseigenschaften in Streifenlängsrichtung. Die Elastizität des Mittelteils, welche die erforderliche Kraft beim Dehnen bzw. die elastische Rückstellkraft bestimmt, wird durch die Eigenschaften der Trägerfolie mitbestimmt. Das auf die Trägerfolie aufkaschierte Spinnvlies weist daher vorzugsweise nur ein Flächengewicht von 10 bis 25 g/m² auf.

Die Trägerfolie ist vorzugsweise eine nach dem Gießfolien- oder Blasfolienverfahren hergestellte Folie aus einem thermoplastischen Elastomer. Hierfür geeignete Polymermischungen beinhalten Polyurethane, Styrol-Blockcopolymere, Polyethylene, Polyethylen-Copolymere, Polypropylen-Copolymere sowie Mischungen aus diesen Polymeren als elastische Komponente. Bedingt durch die Orientierung der Polymere während des Extrusionsprozesses weist die Trägerfolie eine bevorzugte Dehnungsrichtung in einer Richtung auf, während sie in der Querrichtung dazu steif und unelastisch ist. Die Trägerfolie kann auch vernetzte Elastomere, wie z. B. NBR oder EPDM, enthalten.

Die elastische Trägerfolie des Mittelteils hat vorzugsweise ein Flächengewicht zwischen 5 g/m² und 150 g/m². Sie ist eine Monofolie aus einem thermoplastischen Elastomer oder besteht alternativ aus einer mehrschichtigen Coextrusionsfolie, die einen Kern aus einem thermoplastischen Elastomer und eine ein- oder beidseitig angeordnete Haftvermittlerschicht zur Verbesserung der Haftung des angrenzenden Spinnvlieses aufweist. Die Haftvermittlerschicht besitzt zweckmäßigerweise eine hohe Affinität zu Kaschierklebern. Die Dicke der Coextrusionsfolie beträgt beispielsweise 50 µm bis 150 µm mit einem Dickenverhältnis bei dreischichtigem Aufbau von 1:10:1 bis 1:30:1. Der elastische Kern der Coextrusionsfolie ist stets um ein Vielfaches größer als die mitextrudierten Haftvermittlerschichten. Alle Schichten der Coextrusionsfolie können aus elastischen thermoplastischen Polymeren bestehen. Die Haftvermittlerschicht kann durch Additive modifiziert werden, so dass die Trägerfolie nicht verblockt und eine besonders gute Affinität zu Kaschierklebstoffen aufweist. Als Materialien für die Schichten der Coextrusionsfolie können mineralische Füllstoffe sowie Abmischungen von SIS-, SBS-, SEBS- und PU-Polymeren mit Polyolefinen, wie z. B. Polyethylen, Polypropylen, EVA und EPR eingesetzt werden. Die Haftvermittlerschicht kann ferner aus einem polyolefinischen Material bestehen.

Die Trägerfolie und das Spinnvlies des Mittelteils sind vorzugsweise durch einen elastischen Schmelzklebstoff verbunden, wobei der Klebstoffauftrag punktförmig, gitterartig, linienförmig in quer zur Dehnungsrichtung verlaufenden Streifen oder vollflächig erfolgt. Die verwendeten Schmelzklebstoffe sind vorzugsweise elastische Thermoplaste auf Basis von SIS-, SBS- oder SEBS-Polymeren bzw. deren Mischungen. Der Schmelzklebstoff wird vorzugsweise mit Flächengewichten zwischen 2 g/m² und 20 g/m² zwischen der elastischen Trägerfolie und dem Spinnvlies appliziert. Wenn der Schmelzklebstoff in Linien auftragen wird, kann der Abstand der Klebstofflinien 0,2 mm bis 5 mm, vorzugsweise 0,5 mm bis 2 mm, betragen. Die Breite der Klebstoffspur beträgt beispielsweise 0,3 mm bis 2 mm, besonders bevorzugt 0,5 mm bis 1,5 mm.

Bei allen zuvor beschriebenen Ausführungen beträgt der nichtelastische Filamentanteil innerhalb des Spinnvlieses bis zu 60 Gew.-%, vorzugsweise etwa 20 Gew.-%, jeweils bezogen auf das Material des Spinnvlieses.

Die unelastischen Endteile und Anschlussteile des Verschlussstreifens bestehen gemäß einer bevorzugten Ausführung der Erfindung aus einem Vlies aus polyolefinischen Fasern, insbesondere aus Polypropylenfasern. Die Endteile und Anschlussteile sind zweckmäßig durch Verkleben und/oder Verschweißen mit dem Mittelteil verbunden. Eine besonders vorteilhafte Ausführung sieht vor, dass die Endteile und Anschlussteile mit dem Mittelteil verklebt sind und dass zusätzlich eine Punktverschweißung, vorzugsweise durch Ultraschall, vorgesehen ist.

Im Folgenden wird die Erfindung anhand einer lediglich ein Ausführungsbeispiel darstellenden Zeichnung ausführlich erläutert. Es zeigen schematisch
- **Fig. 1a und 1b**: einen erfindungsgemäßen Verschlussstreifen im ungedehnten sowie im gedehnten Zustand;
- **Fig. 2**: eine weitere Ausgestaltung des Verschlussstreifens;
- **Fig. 3a und 3b**: ein Filament, aus dem das Spinnvlies des elastischen Mittelteils des Verschlussstreifens gefertigt ist;
- **Fig. 4**: ein Dehnungs-Spannungs-Diagramm des Verschlussstreifens.

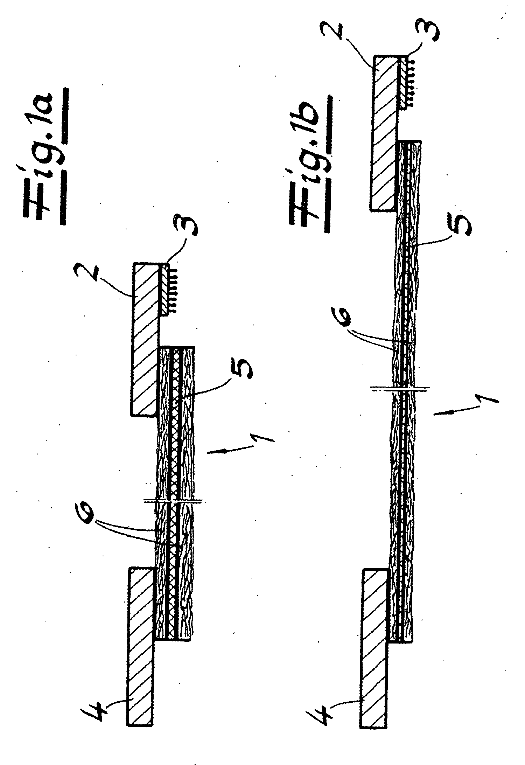

Die Fig. 1a und 1b zeigen einen Verschlussstreifen für einen elastischen Windelverschluss. Der Verschlussstreifen besteht aus einem Mittelteil 1, das beim Ziehen des Verschlussstreifens in Streifenlängsrichtung elastisch ist, einem unelastischen Endteil 2 mit einem Hakenelement 3 und einem unelastischen Anschlussteil 4 zur Befestigung an einer Windel. Das Endteil 2 und das Anschlussteil 4 sind an dem Mittelteil 1 befestigt. Die Streifenlängsrichtung erstreckt sich vom Anschlussteil 4 zum Endteil 2.

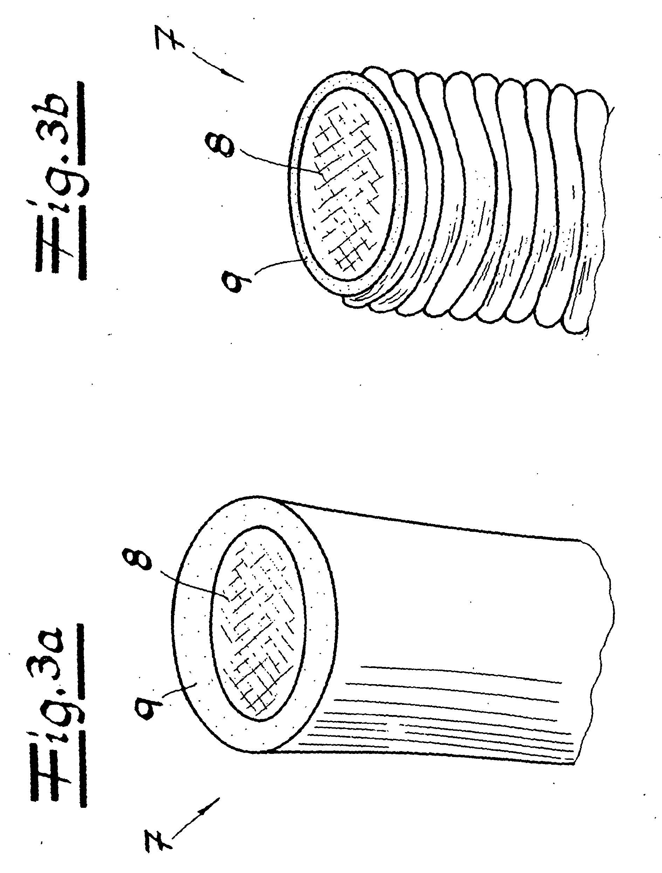

Das Mittelteil 1 des Verschlussstreifens besteht in dem in Fig. 1a und 1b dargestellten Ausführungsbeispiel aus einer elastischen Trägerfolie 5 und mindestens einer aufkaschierten Schicht aus einem elastischen Spinnvlies 6. Die Trägerfolie 5 weist ebenso wie das Spinnvlies 6 bevorzugte Dehnungseigenschaften in Streifenlängsrichtung auf. Das auf die Trägerfolie 5 aufkaschierte Spinnvlies 6 besteht aus Filamenten 7, die entsprechend der Darstellung in Fig. 3b einen Filamentkern 8 aus einem thermoplastischen Elastomer und einen durch Dehnung des Spinnvlieses verstreckten Filamentmantel 9 aus einem nichtelastischen thermoplastischen Polymer aufweisen. Das Flächengewicht des auf die Trägerfolie aufkaschierten Spinnvlieses 6 beträgt vorzugsweise 10 g/m² bis 25 g/m².

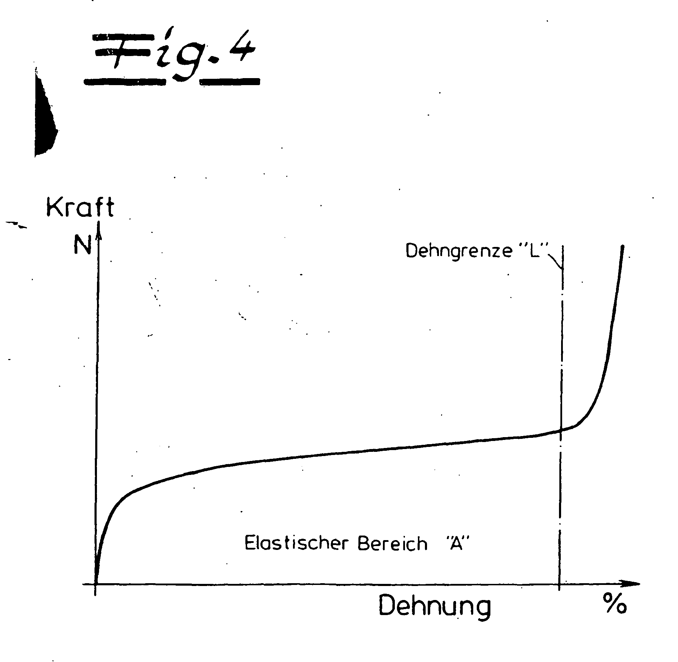

Das auf dem unelastischen Endteil 2 angeordnete Hakenelement 3 weist männliche Verschlusselemente auf, die mit weiblichen Verschlusselementen einen Klettverschluss bilden. Die weiblichen Verschlusselemente sind im vorderen Bündchenbereich einer nicht dargestellten Windel befestigt. Einer vergleichenden Betrachtung der Fig. 1 a und 1b ist zu entnehmen, dass das Mittelteil 1 des Verschlussstreifens in der dargestellten bevorzugten Dehnungsrichtung elastisch dehnbar ist. Das Material des Mittelteils 1 erlaubt beispielsweise eine elastische Auslängung um 30 % bis 150 % der Ursprungslänge, wobei das Erreichen der elastischen Dehngrenze über einen steilen Kraftanstieg definiert ist. Das Dehnungsverhalten des Materials ist in Fig. 4 dargestellt. In der Fig. 4 ist die zur Dehnung erforderliche Zugkraft über die Dehnung (Auslängung bezogen auf die Ursprungslänge) aufgetragen. Der Fig. 4 entnimmt man, dass das Mittelteil 1 des Verschlussstreifens bis zur Dehngrenze mit geringer Kraft gedehnt werden kann. Über diesen Verdehnungsbereich stellt sich das Mittelteil 1 bei Entlastung elastisch zurück. Bei Erreichen der im Material festgelegten Dehngrenze ist ein starker Anstieg der zur weiteren Verdehnung nötigen Kraft festzustellen. Dieser Bereich wird deutlich als nichtelastischer Bereich wahrgenommen.

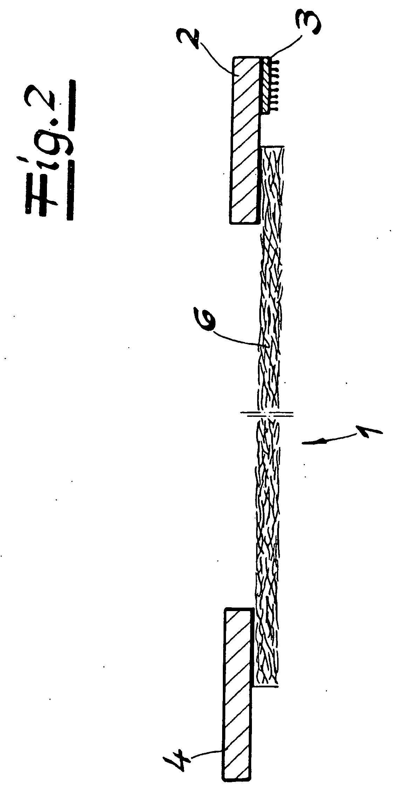

Im Ausführungsbeispiel der Fig. 2 ist das Mittelteil 1 ein einschichtiges Element und besteht ausschließlich aus einem elastischen Spinnvlies 6, welches ein Flächengewicht zwischen 80 g/m² und 200 g/m² aufweist. Das Dehnungsverhalten des Materials entspricht qualitativ der Fig. 3. Es lässt sich mit geringer Kraft elastisch bis zu einer Dehngrenze dehnen, die durch einen steilen Kraftanstieg definiert ist und bei der Handhabung des Streifens deutlich als nichtelastischer Bereich wahrgenommen wird.

Das elastische Spinnvlies 6 besteht aus Filamenten 7, die einen Filamentkern 8 aus einem thermoplastischen Elastomer und einen Filamentmantel 9 aus einem nichtelastischen thermoplastischen Polymer aufweisen. Das Spinnvlies 6 wird durch eine Vorverstreckung modifiziert, durch welche sich die Struktur der Filamente ändert. Dies wird aus einer vergleichenden Betrachtung der Fig. 3a und 3b deutlich. Vor der Verstreckung weisen die Filamente 7 des Spinnvlieses die in Fig. 3a dargestellte Geometrie auf. Durch Vordehnung des Spinnvlieses wird der Filamentmantel 9 der Filamente plastisch deformiert, während der Filamentkern 8 lediglich eine reversible elastische Verformung erfährt. Nach der Vordehnung zieht sich das Filament 7 wieder zusammen und besitzt dann die in Fig. 3b dargestellte Kontur. Aufgrund der plastischen Deformation des nichtelastischen Filamentmantels 9 weist dieser nun eine gewellte Struktur auf. Sofern das Filament 7 im Gebrauch gedehnt wird, richtet sich der nichtelastische Filamentmantel 9 zunächst entlang des elastischen Filamentkernes 8 aus und setzt der Dehnung praktisch keinen Widerstand entgegen. Wenn das Filament 7 durch die Dehnung wieder eine ungewellte Kontur erreicht, welche im Prinzip der Darstellung in Fig. 3a entspricht, ist die elastische Dehngrenze des Faservlieses erreicht. Eine Dehnung über diese Dehngrenze hinaus kann nur erfolgen, wenn der Filamentmantel 9 eine weitere Kaltverstreckung erfährt. Diese zweite Kaltverstreckung ist mit einem steilen Anstieg der zur weiteren Dehnung erforderlichen Kraft verbunden. In diesem Bereich weist das Spinnvlies daher keine elastischen Eigenschaften mehr auf. Dies wird insbesondere anhand der Fig. 4 deutlich, in der die zur Dehnung erforderliche Zugkraft eines erfindungsgemäßen Verschlussstreifens über die Dehnung aufgetragen ist. Im ungedehnten Zustand weisen die Filamente 7 des Mittelteils die in Fig. 3b dargestellte Kontur auf. Im elastischen Dehnungsbereich "A" verläuft die Kurve über einen großen Bereich hinweg verhältnismäßig flach, so dass zur Dehnung des Materials in diesem Bereich nur vergleichsweise geringe Kräfte erforderlich sind. Beim Erreichen der oberen Grenze des elastischen Bereiches besitzen die Filamente 7 eine Kontur, die sie auch während der maximalen Dehnung bei der Vorverstreckung besaßen. Bei einer Dehnung über die elastische Dehngrenze "L" hinaus steigt die hierzu erforderliche Kraft steil nach oben an, die mit einer weiteren Kaltverstreckung des nichtelastischen Filamentmantels 9 einhergeht. Durch die Vorverstreckung der Filamente 7 kann somit die Größe des elastischen Bereiches "A" gezielt eingestellt werden.

Die unelastischen End- und Anschlussteile 2, 4 des Verschlussstreifens bestehen aus einem Vlies aus polyolefinischen Fasern, vorzugsweise aus Polypropylenfasern. Das Endteil 2 und das Anschlussteil 4 sind auf das Mittelteil 1 des Verschlussstreifens aufgeklebt worden und zusätzlich durch Punktschweißungen, vorzugsweise durch Ultraschall, mit dem Mittelteil 1 verbunden.

## Patentansprüche

1. Verschlussstreifen für Windeln mit
einem Mittelteil (1), der beim Ziehen des Verschlussstreifens in Streifenlängsrichtung elastisch ist,
einem unelastischen Endteil (2) mit einem Hakenelement (3) und
einem unelastischen Anschlussteil (4) zur Befestigung an einer Windel,
wobei das Endteil (2) und das Anschlussteil (4) an dem Mittelteil (1) befestigt sind und wobei die Streifenlängsrichtung sich vom Anschlussteil (4) zum Endteil (2) erstreckt, **dadurch gekennzeichnet, dass** das Mittelteil (1) eine Schicht aus einem elastischen Spinnvlies (6) aufweist, welches bevorzugte Dehnungseigenschaften in Streifenlängsrichtung besitzt, wobei das Spinnvlies (6) aus Filamenten (7) besteht, die einen Filamentkern (8) aus einem thermoplastischen Elastomer und einen durch Dehnung des Spinnvlieses verstreckten Filamentmantel (9) aus einem nichtelastischen thermoplastischen Polymer aufweisen, und wobei das Mittelteil (1) bis zu einer durch die Vorverstreckung der nichtelastischen Filamentanteile des Spinnvlieses festgelegten Dehngrenze elastisch dehnbar ist und die Dehngrenze durch einen starken Anstieg der zur weiteren Dehnung benötigten Kraft wahrnehmbar ist.

2. Verschlussstreifen nach Anspruch 1, **dadurch gekennzeichnet, dass** das Mittelteil (1) als einschichtiges Element ausschließlich aus dem Spinnvlies (6) besteht.

3. Verschlussstreifen nach Anspruch 1, **dadurch gekennzeichnet, dass** das Spinnvlies (6) ein Flächengewicht zwischen 80 g/m² und 200 g/m² aufweist.

4. Verschlussstreifen nach Anspruch 1, **dadurch gekennzeichnet, dass** das Mittelteil (1) eine elastische Trägerfolie (5) aufweist, auf die das Spinnvlies (6) ein- oder beidseitig aufkaschiert ist.

5. Verschlussstreifen nach Anspruch 4, **dadurch gekennzeichnet, dass** das auf die Trägerfolie (5) aufkaschierte Spinnvlies ein Flächengewicht von 10 g/m² bis 25 g/m² aufweist.

6. Verschlussstreifen nach Anspruch 4 oder 5, **dadurch gekennzeichnet, dass** die Trägerfolie (5) des Mittelteils (1) eine Monofolie aus einem thermoplastischen Elastomer ist oder aus einer mehrschichtigen Coextrusionsfolie besteht, die einen Kern aus einem thermoplastischen Elastomer und eine ein- oder beidseitig angeordnete Haftvermittlerschicht zur Verbesserung der Haftung des angrenzenden Spinnvlieses (6) aufweist.

7. Verschlussstreifen nach einem der Ansprüche 4 bis 6, **dadurch gekennzeichnet, dass** die Trägerfolie (5) und das Spinnvlies (6) durch einen elastischen Schmelzklebstoff verbunden sind, wobei der Klebstoffauftrag punktförmig, gitterartig, linienförmig in quer zur Dehnungsrichtung verlaufenden Streifen oder vollflächig erfolgt.

8. Verschlussstreifen nach einem der Ansprüche 4 bis 7, **dadurch gekennzeichnet, dass** die elastische Trägerfolie (5) des Mittelteils (1) ein Flächengewicht zwischen 5 g/m² und 150 g/m² aufweist.

9. Verschlussstreifen nach einem der Ansprüche 1 bis 8, **dadurch gekennzeichnet, dass** der nichtelastische Filamentanteil innerhalb des Spinnvlieses bis zu 60 Gew.%, vorzugsweise etwa 20 Gew.-%, beträgt.

10. Verschlussstreifen nach einem der Ansprüche 1 bis 9, **dadurch gekennzeichnet, dass** die unelastischen End- und Anschlussteile (2, 4) aus einem Vlies aus polyolefinischen Fasern, vorzugsweise aus Polypropylenfasern, bestehen.

11. Verschlussstreifen nach einem der Ansprüche 1 bis 10, **dadurch gekennzeichnet, dass** das Endteil (2) und das Anschlussteil (4) durch Verkleben und/oder Verschweißen mit dem Mittelteil (1) verbunden sind.

## Claims

1. A sealing strip for diapers comprising
a center part (1), which is elastic in response to pulling the sealing strip in longitudinal strip direction,
an inelastic end part (2) comprising a hook element (3) and
an inelastic connecting part (4) for fastening to a diaper,
wherein the end part (2) and the connecting part (4) are fastened to the center part (1) and wherein the longitudinal strip direction extends from the connecting part (4) to the end part (2), **characterized in that** the center part (1) encompasses a layer of an elastic spunbonded fabric (6), which has preferred expansion characteristics in the longitudinal strip direction, wherein the spunbonded fabric (6) consists of filaments (7), which encompass a filament core (8) made of a thermoplastic elastomer and a filament jacket (9), which is made of an inelastic thermoplastic polymer and which is stretched by expanding the spunbonded fabric, and wherein the center part (1) can be elastically expanded up to an elastic limit, which is determined by the pre-stretching of the inelastic filament portions of the spunbonded fabric and **in that** the elastic limit can be perceived by a high increase of the force required for the further expansion.

2. The sealing strip according to claim 1, **characterized in that** the center part (1), as a one-layer element, consists exclusively of the spunbonded fabric (6).

3. The sealing strip according to claim 1, **characterized in that** the spunbonded fabric (6) encompasses a mass per unit area of between 80 g/m² and 200 g/m².

4. The sealing strip according to claim 1, **characterized in that** the center part (1) encompasses an elastic supporting film (5), onto which the spunbonded fabric (6) is laminated on one or both sides.

5. The sealing strip according to claim 4, **characterized in that** the spunbonded fabric, which is laminated onto the supporting film (5) encompasses a mass per unit area of from 10 g/m² to 25 g/m².

6. The sealing strip according to claim 4 or 5, **characterized in that** the supporting film (5) of the center part (1) is a mono film made of a thermoplastic elastomer or consists of a multi-layer coextrusion film, which encompasses a core made of a thermoplastic elastomer and an adhesive layer, which is arranged on one or both sides, for the purpose of improving the adhesion of the adjacent spunbonded fabric (6).

7. The sealing strip according to one of claims 4 to 6, **characterized in that** the supporting film (5) and the spunbonded fabric (6) are connected by means of an elastic hot melt adhesive, wherein the adhesive application takes place in a punctiform manner, in a lattice-like manner, in a linear manner in strips running at right angles to the expansion direction or in a holohedral manner.

8. The sealing strip according to one of claims 4 to 7, **characterized in that** the elastic supporting film (5) of the center part (1) encompasses a mass per unit area of between 5 g/m² and 150 g/m².

9. The sealing strip according to one of claims 1 to 8, **characterized in that** the inelastic filament portion within the spunbonded fabric is up to 60 weight %, preferably approximately 20 weight %.

10. The sealing strip according to one of claims 1 to 9, **characterized in that** the inelastic end and connecting parts (2, 4) consist of a non-woven material of polyolefinic fibers, preferably of polypropylene fibers.

11. The sealing strip according to one of claims 1 to 10, **characterized in that** the end part (2) and the connecting part (4) are connected to the center part (1) by means of adhesion and/or fusion.

## Revendications

1. Patte de fermeture pour couches avec
une partie centrale (1), qui à la traction de la patte de fermeture est élastique en direction longitudinale de la patte,
une partie d'extrémité non élastique (2) avec un élément à crochets (3) et une partie de raccordement (4) destinée à être fixée sur une couche,
la partie d'extrémité (2) et la partie de raccordement (4) étant fixées sur la partie centrale (1) et la direction longitudinale de la patte s'étendant de la partie de raccordement (4) vers la partie d'extrémité (2), **caractérisée en ce que** la partie centrale (1) comporte une couche en un voile non tissé élastique (6) présentant de préférence des caractéristiques extensibles en direction longitudinale de la patte, le voile non tissé (6) consistant dans des filaments (7), comportant un coeur de filament (8) en un élastomère thermoplastique et dans une enveloppe de filament (9) étirée par extension du voile non tissé en un polymère thermoplastique non élastique et la partie centrale (1) étant élastiquement extensible jusqu'à une limite d'extension prédéfinie par le pré-étirage des parties non élastiques des filaments du voile non tissé et la limite d'extension étant perceptible par une forte augmentation de la force nécessaire pour l'extension ultérieure.

2. Patte de fermeture selon la revendication 1, **caractérisée en ce que**, en tant qu'élément monocouche, la partie centrale (1) consiste exclusivement dans le voile non tissé (6).

3. Patte de fermeture selon la revendication 1, **caractérisée en ce que** le voile non tissé (6) présente un grammage compris entre 80 g/m² et 200 g/m².

4. Patte de fermeture selon la revendication 1, **caractérisée en ce que** la partie centrale (1) comporte un film support élastique (5) sur lequel le voile non tissé (6) est unilatéralement ou bilatéralement contrecollé.

5. Patte de fermeture selon la revendication 4, **caractérisée en ce que** le voile non tissé contrecollé sur le film support (5) présente un grammage de 10 g/m² à 25 g/m².

6. Patte de fermeture selon la revendication 4 ou 5, **caractérisée en ce que** le film support (5) de la partie centrale (1) est un mono-film en un élastomère thermoplastique ou consiste dans un film multicouches co-extrudé comportant un coeur en un élastomère thermoplastique et une couche d'agent adhésif disposée unilatéralement et/ou bilatéralement pour améliorer l'adhérence du voile non tissé adjacent (6).

7. Patte de fermeture selon l'une quelconque des revendications 4 à 6, **caractérisée en ce que** le film support (5) et le voile non tissé (6) sont assemblés par une colle fusible, l'application de colle s'effectuant en mode ponctuel, en grille, sous forme linéaire, par bandes s'étendant à la transversale de la direction d'extension ou sur toute la surface.

8. Patte de fermeture selon l'une quelconque des revendications 4 à 7, **caractérisée en ce que** le film support élastique (5) de la partie centrale (1) présente un grammage compris entre 5 g/m² et 150 g/m².

9. Patte de fermeture selon l'une quelconque des revendications 1 à 8, **caractérisée en ce que** la fraction de film non élastique à l'intérieur du voile non tissé s'élève à de jusqu'à 60 % en poids, de préférence à environ 20 % en poids.

10. Patte de fermeture selon l'une quelconque des revendications 1 à 9, **caractérisée en ce que** les parties d'extrémité et de raccordement non élastiques (2, 4) consistent dans un voile en fibres polyoléfines, de préférence en fibres de polypropylène.

11. Patte de fermeture selon l'une quelconque des revendications 1 à 10, **caractérisée en ce que** la partie d'extrémité (2) et la partie de raccordement (4) sont assemblées par collage et/ou par soudage sur la partie centrale (1).
